(19) Europäisches Patentamt / European Patent Office / Office européen des brevets

(11) **EP 2 311 370 B1**

(12) **FASCICULE DE BREVET EUROPEEN**

(45) Date de publication et mention
de la délivrance du brevet:
**13.03.2013 Bulletin 2013/11**

(51) Int Cl.:
*A61B 5/06* (2006.01)    *G01V 3/08* (2006.01)

(21) Numéro de dépôt: **10187336.2**

(22) Date de dépôt: **12.10.2010**

(54) **Procédé de détection et détecteur d'un perturbateur magnétique, procédé et systeme de localisation d'un objet, support d'enregistrement pour ces procédés**

Erkennungsverfahren und Detektor für ein magnetisches Störelement, Verfahren und System zur Lokalisierung eines Objekts und Datenträger für diese Verfahren

Magnetic disturber detection method and detector, object-localizing method and system, recording medium for these methods

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.10.2009 FR 0957205**

(43) Date de publication de la demande:
**20.04.2011 Bulletin 2011/16**

(73) Titulaire: **Commissariat à l'Énergie Atomique et aux Énergies Alternatives
75015 Paris (FR)**

(72) Inventeurs:
• **Billeres, Malvina
38000 GRENOBLE (FR)**
• **Blanpain, Roland
38380 ENTRE-DEUX-GUIERS (FR)**

(74) Mandataire: **Colombo, Michel et al
Brevinnov
324, rue Garibaldi
69007 Lyon (FR)**

(56) Documents cités:
**EP-A1- 1 502 544        US-A1- 2007 055 125
US-A1- 2007 167 722    US-B1- 6 841 994**

**Description**

L'invention concerne un procédé de détection et un détecteur d'un perturbateur magnétique du champ magnétique émis par un émetteur au moins triaxe de champ magnétique. L'invention concerne également un procédé et un système de localisation d'un objet et un support d'enregistrement d'informations pour mettre en oeuvre ces procédés.

Un perturbateur magnétique est ici défini comme étant tout objet qui altère ou déforme les lignes de champ d'un champ magnétique émis à proximité. Par exemple, le perturbateur peut être une pièce conductrice. Dans ce cas, l'altération des lignes de champ magnétique est due à l'apparition de courants de Foucault (« Eddy Current ») dans la pièce conductrice. La pièce conductrice est par exemple une pièce métallique. Le perturbateur peut également être une pièce magnétique telle qu'une pièce paramagnétique ou ferromagnétique.

Un émetteur triaxe de champ magnétique émet un champ magnétique le long de trois axes d'émission non colinéaires entre eux. Par exemple, un tel émetteur est formé de plusieurs sources monoaxes de champ magnétique alignées, respectivement, sur chacun des axes d'émission de l'émetteur.

Une source monoaxe de champ magnétique est une source qui émet préférentiellement le champ magnétique le long d'un seul axe. Par exemple, une bobine dont les spires sont enroulées autour d'un même axe est une source monoaxe de champ magnétique et l'axe d'émission préférentiel est confondu avec l'axe d'enroulement des spires.

De façon similaire, un émetteur au moins triaxe est un émetteur qui émet des champs magnétiques le long de plus de trois axes non colinéaires.

On définit également un capteur triaxe de champ magnétique comme étant un capteur apte à mesurer la direction du champ magnétique. Typiquement, à cet effet, ces capteurs mesurent l'amplitude de la projection du champ magnétique sur trois axes de mesure non colinéaires entre eux. Ainsi, ces capteurs permettent de mesurer la direction du champ magnétique et, généralement, en plus l'amplitude de ce champ magnétique.

Des procédés connus de détection d'un perturbateur magnétique comportent la mesure du champ magnétique émis par l'émetteur par au moins deux capteurs triaxes placés à des positions différentes connues.

Les procédés de détection de perturbateur magnétique sont particulièrement utilisés au sein de procédés de localisation d'un objet à l'aide d'un système magnétique. En effet, si le champ magnétique mesuré utilisé pour localiser un objet est perturbé, la localisation de l'objet est alors erronée. Ceci peut avoir des conséquences particulièrement néfastes lorsque le procédé de localisation est utilisé en médecine pour localiser une sonde ou un cathéter à l'intérieur d'un corps humain. En effet, pour de telles applications, la fiabilité de la localisation de la sonde est très importante. Or, dans le milieu médical, il existe de nombreux perturbateurs magnétiques susceptibles de fausser la localisation. Par exemple, le perturbateur magnétique peut être une table d'opération, le scalpel d'un chirurgien, l'armature métallique d'un autre appareil placé à proximité du patient, ...etc.

Plusieurs méthodes ont déjà été proposées pour détecter les perturbateurs (voir par exemple EP 1 502 544 ou EP 0 993 804). Ces méthodes font appel à des calculs complexes.

De l'état de la technique est également connu de US 6 841 994 B1.

L'invention vise à remédier à ce problème en détectant simplement la présence d'un perturbateur du champ magnétique émis.

Elle a donc pour objet un procédé de détection d'un perturbateur du champ magnétique émis comprenant :

- pour chaque capteur, la détermination, à partir du champ magnétique mesuré par ce capteur, des coordonnées d'un vecteur directeur colinéaire à un axe passant par le centre géométrique de l'émetteur et le centre géométrique de ce capteur, le centre géométrique de l'émetteur étant le point où se situe une source ponctuelle de champ magnétique qui modélise cet émetteur et le capteur étant modélisable par un transducteur ponctuel situé en un point au niveau duquel est mesuré le champ magnétique et constituant le centre géométrique de ce capteur,
- la vérification que la plus petite distance entre les axes colinéaires chacun à l'un des vecteurs directeurs est inférieure à une limite prédéterminée, et
- dans la négative, le signalement de la présence d'un perturbateur magnétique et, dans le cas contraire, l'absence de ce signalement.

Le procédé ci-dessus est basé sur le fait qu'en absence de perturbateur et dans le cas idéal, les axes colinéaires aux vecteurs directeurs doivent se couper en un point E correspondant à la position de l'émetteur. La présence d'un perturbateur magnétique modifie la direction d'un ou plusieurs vecteurs directeurs. Par conséquent, au moins l'un des axes colinéaires à ces vecteurs directeurs ne coupe plus les autres axes. Dans ce cas, la plus petite distance $\underline{d}$ entre au moins deux de ces axes est non nulle. Dès lors, si cette distance $\underline{d}$ dépasse la limite prédéterminée, cela indique la présence d'un perturbateur.

Ce procédé est simple à mettre en oeuvre car le calcul des vecteurs directeurs nécessite seulement quelques multiplications et additions. Il peut donc s'exécuter très rapidement et être appliqué en « temps réel » même si les capteurs et l'émetteur se déplacent rapidement l'un par rapport à l'autre. De plus, il est particulièrement sensible. En effet, une légère perturbation de la direction d'un des vecteurs directeurs peut se traduire par une modification importante de la distance minimale d.

Enfin, il ne nécessite pas ou pratiquement pas de calibration initiale. Seule la position des capteurs l'un par rapport à l'autre doit être connue au préalable.

Les modes de réalisation de ce procédé peuvent comporter une ou plusieurs des caractéristiques suivantes :

■ l'étape de vérification est au moins réalisée pour des premier et second vecteurs directeurs $\vec{u}_1$, $\vec{u}_2$ et comprend :

- le calcul des coordonnées d'un premier vecteur $\vec{n}_1$ normal au vecteur directeur $\vec{u}_1$ et à un axe D passant par les centres géométriques des deux capteurs utilisés pour déterminer les vecteurs directeurs $\vec{u}_1$ et $\vec{u}_2$,
- le calcul des coordonnées d'un second vecteur $\vec{n}_2$ normal au vecteur directeur $\vec{u}_2$ et à l'axe D,
- le calcul de l'angle entre les vecteurs $\vec{n}_1$ et $\vec{n}_2$, et
- la vérification que cet angle est inférieur à un seuil correspondant à la limite prédéterminée au-delà de laquelle le signalement du perturbateur magnétique est déclenché ;

■ l'étape de vérification est au moins réalisée pour des premier et second vecteurs directeurs $\vec{u}_1$, $\vec{u}_2$ et comprend :

- le calcul de la valeur d'un produit mixte, le produit mixte étant défini par la relation suivante : $(\vec{D} \otimes \vec{u}_1) \cdot \vec{u}_2$, où $\otimes$ et '.' sont, respectivement, les opérations produits vectoriel et scalaire et le vecteur D est un vecteur colinéaire à un axe D passant par les centres géométriques des deux capteurs utilisés pour déterminer les vecteurs directeurs $\vec{u}_1$ et $\vec{u}_2$, et
- le contrôle que la valeur du produit mixte est inférieure à un seuil correspondant à la limite prédéterminée au-delà de laquelle le signalement du perturbateur magnétique est déclenché;

■ les positions des capteurs, dans un même repère, sont fixes.

Ces modes de réalisation du procédé de détection présentent en outre l'avantage suivant :

- le calcul de l'angle entre les vecteurs $\vec{n}_1$, et $\vec{n}_2$ accroît la précision de la détection du perturbateur magnétique.

L'invention a également pour objet un procédé de localisation d'un objet dans un repère à l'aide d'au moins un émetteur au moins triaxe de champ magnétique et d'au moins deux capteurs triaxes du champ magnétique émis par l'émetteur, chaque capteur étant solidaire du repère et chaque émetteur étant solidaire de l'objet ou vice versa, ce procédé comportant :

- l'émission d'un champ magnétique par l'émetteur et la mesure de ce champ magnétique par les capteurs,
- la localisation de l'objet dans le repère à partir des mesures réalisées par les capteurs,
- la détection d'un perturbateur du champ magnétique émis par l'émetteur en utilisant les mêmes capteurs que lors de l'étape de localisation et en mettant en oeuvre le procédé de détection ci-dessus.

L'invention a également pour objet un support d'enregistrement d'informations comprenant des instructions pour l'exécution de l'un des procédés ci-dessus, lorsque ces instructions sont exécutées par un calculateur électronique.

L'invention a également pour objet un détecteur de perturbateur magnétique d'un champ magnétique émis par un émetteur au moins triaxe de champ magnétique, ce détecteur comportant :

- au moins deux capteurs triaxes aptes à mesurer le champ magnétique émis par l'émetteur, ces capteurs étant placés à des positions différentes connues, et
- une unité de traitement raccordée aux capteurs pour traiter les mesures du champ magnétique de l'émetteur, cette unité de traitement étant apte :
- pour chaque capteur, à déterminer, à partir du champ magnétique mesuré par ce capteur, des coordonnées d'un vecteur directeur colinéaire à un axe passant par le centre géométrique de l'émetteur et le centre géométrique de ce capteur, le centre géométrique de l'émetteur étant le point où se situe une source ponctuelle de champ magnétique qui modélise cet émetteur et le capteur étant modélisable par un transducteur ponctuel situé en un point au niveau duquel est mesuré le champ magnétique et constituant le centre géométrique de ce capteur,
- à vérifier que la plus petite distance entre les axes colinéaires chacun à un des vecteurs directeurs est inférieure à une limite prédéterminée, et
- dans la négative, à signaler la présence d'un perturbateur magnétique et, dans le cas contraire, à ne pas signaler la présence de ce perturbateur magnétique.

Enfin, l'invention a également pour objet un système de localisation d'un objet dans un repère, ce système comportant :

- au moins un émetteur au moins triaxe de champ magnétique,
- au moins deux capteurs triaxes pour mesurer le champ magnétique émis par cet émetteur, les capteurs étant fixes dans le repère et l'émetteur ou les émetteurs étant solidaires de l'objet ou vice versa,
- un module de localisation apte à localiser l'objet dans le repère à partir des mesures réalisées par les capteurs,
- le détecteur ci-dessus d'un perturbateur du champ magnétique émis par l'émetteur, les capteurs de ce détecteur étant communs avec ceux utilisés par le module de localisation.

L'invention sera mieux comprise à la lecture de la description qui va suivre, donnée uniquement à titre d'exemple non limitatif et faite en se référant aux dessins sur lesquels :

- la figure 1 est une illustration schématique d'un système de localisation d'un objet équipé d'un détecteur de perturbateur magnétique,
- la figure 2 est un organigramme d'un procédé de détection d'un perturbateur magnétique et de localisation d'un objet à l'aide du système de la figure 1,
- la figure 3 est un organigramme d'un autre mode de réalisation d'un procédé de détection d'un perturbateur magnétique et de localisation d'un objet à l'aide du système de la figure 1.

Dans ces figures, les mêmes références sont utilisées pour désigner les mêmes éléments.

Dans la suite de cette description, les caractéristiques et fonctions bien connues de l'homme du métier ne sont pas décrites en détail.

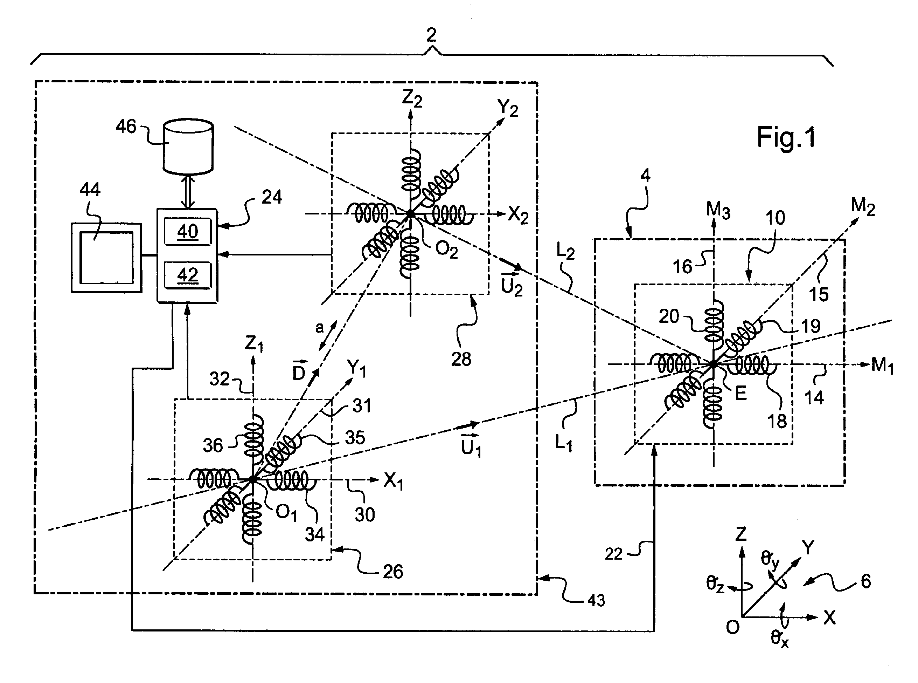

La figure 1 représente un système 2 de localisation d'un objet 4 dans un repère 6. Par exemple, l'objet 4 est une sonde ou un cathéter introduit dans un corps humain. L'objet 4 est ici mobile par rapport au repère 6.

Le repère 6 est un référentiel fixe présentant trois axes orthogonaux X, Y et Z.

La localisation de l'objet 4 dans le repère 6 consiste, par exemple, à trouver sa position x, y, z et son orientation $\theta x$, $\theta y$, $\theta z$. Les angles $\theta x$, $\theta y$ et $\theta z$ représentent l'orientation de l'objet 4, respectivement, autour des axes X, Y et Z.

Pour localiser l'objet 4 dans le repère 6, celui-ci est, par exemple, équipé de plusieurs émetteurs de champ magnétique. Pour simplifier la figure 1, seul un émetteur 10 a été représenté.

L'émetteur 10 est un émetteur triaxe qui émet un champ magnétique selon trois axes non colinéaires 14 à 16. Ici, ces axes 14 à 16 d'émission sont orthogonaux entre eux. Ces axes sont solidaires de l'objet 4. A cet effet, l'émetteur 10 incorpore trois sources monoaxes 18 à 20 correspondant, respectivement, aux moments magnétiques $M_1$, $M_2$ et $M_3$. Chacune de ces sources présente une seule direction d'émission le long de laquelle l'essentiel du champ magnétique est émis. Ici, les directions d'émission des sources 18 à 20 sont confondues, respectivement, avec les axes 14 à 16 sur lesquels sont alignés les moments magnétiques $M_1$, $M_2$ et $M_3$.

Chacune de ces sources 18 à 20 peut être modélisée par une source ponctuelle de champ magnétique. De préférence, les sources 18 à 20 sont agencées de manière à ce que leurs sources ponctuelles respectives occupent exactement la même position dans le repère 6. Cette position est repérée par un point E. Le point E est à l'intersection des axes 14 à 16. Le point où se superposent les sources ponctuelles correspondant aux sources 18 à 20 constitue le centre géométrique de l'émetteur 10. Ici ce centre géométrique est confondu avec le barycentre ou le centre de masse des sources 18 à 20.

Par exemple, chaque source 18 à 20 est constituée d'une seule bobine enroulée autour, respectivement, des axes 14 à 16. Ici, chacune de ces bobines est divisée en deux groupes identiques de spires réparties à part égal de part et d'autre du point E le long de l'axe d'enroulement. Chaque groupe de spires est bobiné dans le même sens le long de l'axe d'enroulement.

L'émetteur 10 est alimenté et commandé par l'intermédiaire d'une liaison filaire souple 22 raccordée à une unité 24 de traitement.

L'unité 24 est également raccordée à plusieurs capteurs triaxes de champ magnétique. Pour simplifier la figure 1, seuls deux capteurs 26 et 28 sont représentés. Chacun de ces capteurs est apte à mesurer la direction et l'amplitude du champ magnétique émis par l'émetteur 10.

Ces capteurs 26 et 28 sont espacés l'un de l'autre par une distance $\underline{a}$.

Le capteur 26 est fixe dans le repère 6. Ce capteur 26 mesure la projection du champ magnétique le long de trois axes orthogonaux 30 à 32. Ici, ces axes 30 à 32 sont colinéaires, respectivement, aux axes $X_1$, $Y_1$ et $Z_1$ d'un repère orthogonal $R_1$ dont le centre $O_1$ est centré sur le capteur 26.

Le capteur 26 est ici composé de trois transducteurs monoaxe 34 à 36. Chaque transducteur 34 à 36 mesure la projection du champ magnétique émis, respectivement, sur les axes 30 à 32.

Par exemple, chacun de ces transducteurs 34 à 36 est constitué d'une seule bobine enroulée autour, respectivement, des axes 30 à 32. Comme pour l'émetteur 10, chacune de ces bobines est divisée en deux groupes identiques de spires réparties de façon symétrique de part et d'autre du point $O_1$ le long de l'axe d'enroulement. Chaque groupe de spires est bobiné dans le même sens le long de l'axe d'enroulement. Ainsi, chacun des transducteurs 30 à 32 est modélisable par un transducteur ponctuel au niveau duquel est mesuré la projection du champ magnétique sur l'axe de mesure. Ici, les trois transducteurs ponctuels sont placés en $O_1$. Le point où se superposent les différents transducteurs ponctuels constitue le centre géométrique du capteur. Ici ce centre géométrique est confondu avec le barycentre ou le centre de masse des transducteurs.

Un tel capteur 26 mesure la direction du champ magnétique émis au niveau du point $O_1$.

Le capteur 28 est par exemple identique au capteur 26 à l'exception du fait que les bobines sont enroulées, respectivement, autour de trois axes $X_2$, $Y_2$ et $Z_2$ d'un repère orthogonal $R_2$ dont l'origine $O_2$ est confondue avec le centre géométrique du capteur 28.

Pour simplifier les calculs, les axes $X_2$, $Y_2$ et $Z_2$ sont parallèles, respectivement, aux axes $X_1$, $Y_1$ et $X_1$.

De préférence, la distance a est au moins deux ou trois fois supérieure à la plus grande dimension de l'un des capteurs. Par exemple, la plus grande dimension du capteur 26 est ici la plus grande longueur d'un des transducteurs 32 à 34.

Les capteurs 26 et 28 sont également éloignés de l'émetteur 10 d'une distance au moins deux ou trois fois supérieure à la plus grande dimension du capteur 26 ou 28. Dans ces conditions, l'émetteur 10 peut être modélisé comme une source ponctuelle de champ magnétique centrée sur le point E.

L'unité 24 comprend un module 40 de localisation de la position de l'émetteur 10 dans le repère 6 à partir des mesures réalisées par les capteurs 26 et 28. Par exemple, le module 40 détermine la position et l'orientation de l'objet 4 en résolvant un système d'équations. Ce système d'équations est obtenu en modélisant les interactions magnétiques entre les sources monoaxes et les transducteurs sans tenir compte de la présence de perturbateur magnétique. Dans ce système d'équations, la position x, y et z et l'orientation θx, θy et θz de l'objet 4 sont les inconnues alors que les valeurs des autres paramètres sont obtenues à partir des mesures réalisées par les capteurs 26 et 28. Plus d'informations sur de tels systèmes d'équations peuvent, par exemple, être trouvées dans la demande de brevet EP 1 502 544.

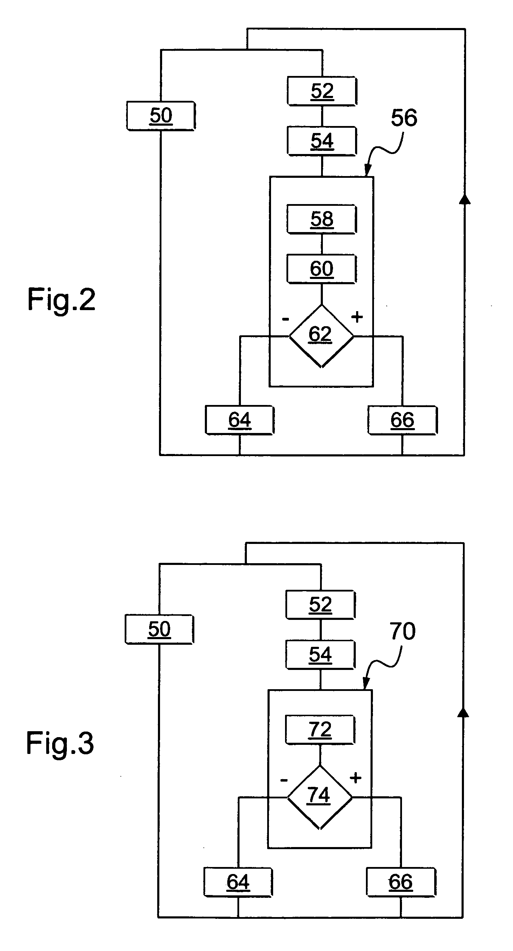

L'unité 24 comprend également un module 42 de détection de la présence d'un perturbateur du champ magnétique émis par l'émetteur 10. Le fonctionnement de ce module 42 sera décrit plus en détail en regard des figures 2 et 3.

La réunion des capteurs 26, 28 et du module 42 forme un détecteur 43 de perturbateur du champ magnétique émis par l'émetteur 10.

L'unité 24 est également raccordée à une interface homme-machine 44 permettant, par exemple, d'indiquer à un opérateur qu'un perturbateur magnétique a été détecté ou d'indiquer la position de l'objet 4.

L'unité 24 est réalisée à partir d'un calculateur électronique programmable apte à exécuter des instructions enregistrées sur un support d'enregistrement d'informations. A cet effet, l'unité 24 est raccordée à une mémoire 46 comportant les instructions nécessaires pour l'exécution du procédé de la figure 2 ou 3.

Le fonctionnement du système 2 va maintenant être décrit plus en détail en regard du procédé de la figure 2.

Lors d'une étape 50, l'émetteur 10 émet, par exemple séquentiellement, un champ magnétique le long de chacun des axes 14 à 16.

En parallèle, lors d'une étape 52, chacun des capteurs 26 et 28 mesure le champ magnétique émis par l'émetteur 10.

Ensuite, lors d'une étape 54, le module 42 de détection détermine des vecteurs directeurs $\vec{u}_1$ et $\vec{u}_2$ dirigés, respectivement, du point $O_1$ vers le point E ou vice versa et du point $O_2$ vers le point E ou vice versa. Sur la figure 1, ces vecteurs sont représentés comme étant tous les deux dirigés vers le point E.

Dans les conditions de fonctionnement décrites précédemment, l'émetteur 10 émet un champ approximé à un champ dipolaire. Dans ces conditions, le champ magnétique émis par une source monoaxe i et mesuré par le capteur triaxe 26 est donné par la relation suivante :

$$H_i = 100 \cdot \frac{3 * u_1 * u_1^T - I}{R^3} M_i$$

où :

- l'indice i est l'identifiant de l'une des sources monoaxes 18 à 20,
- $M_i$ est la matrice colonne associée au moment magnétique de la source monoaxe i de l'émetteur 10,
- « I » est la matrice identité,
- $u_1$ est une matrice colonne contenant les coordonnées d'un vecteur directeur $\vec{u}_1$ exprimées dans le repère $R_1$,
- R est la distance entre l'émetteur 10 et le capteur 26, et

- $H_i$ est la matrice colonne contenant les coordonnées du champ magnétique mesuré le long des trois axes 30 à 32 du capteur 26,
- le symbole « $T$ » représente l'opération transposée.

Dans la suite de cette description, on note $H_1$, $H_2$ et $H_3$ les matrices colonnes contenant les mesures le long des axes 30 à 32 du champ magnétique émis, respectivement, par les sources monoaxe 18, 19 et 20. Avec ces notations, les coordonnées du vecteur $\vec{u}_1$ dans le repère $R_1$ sont obtenues à l'aide de la relation suivante :

$$\vec{u_1} = \left[ H_1 * \left( H_2^T * H_3 \right) - H_3 * \left( H_1^T * H_2 \right) \right] \otimes \left[ H_2 * \left( H_3^T * H_1 \right) - H_3 * \left( H_1^T * H_2 \right) \right]$$

De façon similaire, lors de l'étape 54, le module 42 détermine également les coordonnées d'un vecteur directeur $\vec{u}_2$ pointant du point $O_2$ vers le point E.

Ici, on note $L_1$ et $L_2$ les axes colinéaires aux vecteurs directeurs $\vec{u1}$ et $\vec{u}_2$.

Puisque les capteurs 26 et 28 mesurent le champ magnétique émis par le même émetteur, en absence de perturbation, les axes $L_1$ et $L_2$ doivent se couper au niveau du point E. Toutefois, en pratique, à cause du bruit sur la mesure et d'erreur de calcul, les axes $L_1$ et $L_2$ ne se coupent pas mais passent à proximité l'un de l'autre au niveau du point E. Dans ces conditions, la plus petite distance $\underline{d}$ entre les axes $L_1$ et $L_2$ est faible. A l'inverse, si un perturbateur magnétique est présent, il modifie sensiblement la direction du vecteur $\vec{u}_1$ ou $\vec{u}_2$ de sorte que la plus petite distance $\underline{d}$ devient importante.

Ainsi, lors d'une étape 56, on vérifie que la distance d entre les axes $L_1$ et $L_2$ est inférieure à une limite prédéterminée.

Par exemple, pour cela, lors d'une opération 58, le module 42 calcule les coordonnées dans le repère $R_1$ d'un vecteur $\vec{n}_1$ normal au vecteur $\vec{u}_1$ et à un vecteur $\vec{D}$. Le vecteur $\vec{D}$ est un vecteur colinéaire à un axe D passant par les points $O_1$ et $O_2$. Les coordonnées de ce vecteur $\vec{D}$ dans le repère $R_1$ peuvent facilement être déterminées à partir des coordonnées des points $O_1$ et $O_2$ dans le repère 6 puisque la position des capteurs 26 et 28 est ici fixe.

Par exemple, les coordonnées du vecteur $\vec{n}_1$ sont calculées à l'aide de la relation suivante :

$$\vec{n_1} = \vec{D} \otimes \vec{u_1}$$

où :

- $\otimes$ est l'opération produit vectoriel.

De façon similaire, lors de l'opération 58, les coordonnées d'un vecteur $\vec{n}_2$ normal au vecteur $\vec{u}_2$ et au vecteur $\vec{D}$ sont calculées. Les coordonnées du vecteur $\vec{n}_2$ sont par exemple exprimées dans le repère $R_1$.

Ensuite, lors d'une opération 60, l'angle $\theta$ entre les vecteurs $\vec{n}_1$, et $\vec{n}_2$ est calculé. Etant donné qu'ici les axes des repères $R_1$ et $R_2$ sont parallèles, cet angle $\theta$ peut être calculé directement à l'aide de la relation suivante :

$$\theta = a\cos\left( \frac{\vec{n_1} . \vec{n_2}}{\|\vec{n_1}\| * \|\vec{n_2}\|} \right)$$

où :

- « acos » représente l'opération arc cosinus,
- le symbole « • » représente le produit scalaire,
- le symbole « * » représente la multiplication entre deux valeurs,
- le symbole « ‖...‖ » représente la norme euclidienne d'un vecteur.

Selon les directions des vecteurs $\vec{u}_1$ et $\vec{u}_2$, cet angle $\theta$ doit être proche de 0° ou proche de 180° en cas d'absence de perturbateur magnétique. On suppose ici que les directions des vecteurs $\vec{u}_1$ et $\vec{u}_2$ sont telles que l'angle $\theta$ doit être proche de 0° en cas d'absence de perturbateur magnétique.

Ensuite, lors d'une opération 62, l'angle $\theta$ est comparé à un seuil $S_1$ pour contrôler que la distance d est inférieure à une limite prédéterminée.

Si ce seuil $S_1$ est dépassé, alors cela signifie qu'un perturbateur magnétique est présent. On procède alors à une étape 64 lors de laquelle la présence de ce perturbateur magnétique est signalée. Par exemple, cette présence est signalée par l'intermédiaire de l'interface homme-machine 44. Le signalement peut aussi consister simplement à prendre en compte cette information dans les traitements réalisés par l'unité 24 sans nécessairement en informer l'utilisateur.

Dans le cas contraire, si l'angle θ est inférieur au seuil $S_1$, alors cela signifie qu'il n'y a pas de perturbateur magnétique. On procède alors à une étape 66 lors de laquelle, par exemple, le module 40 détermine la localisation de l'émetteur 10 dans le repère 6 à partir des mesures réalisées par les capteurs 26 et 28.

A l'issue des étapes 50, 64 et 66, le procédé retourne aux étapes 50 et 52.

Le procédé de la figure 3 est identique au procédé de la figure 2 à l'exception que l'étape 56 est remplacée par une étape 70. Au début de cette étape 70, lors d'une opération 72, le produit mixte P entre les vecteurs $\vec{u_1}$ et $\vec{u_2}$ est calculé selon la relation suivante :

$$P = (\vec{D} \otimes \vec{u_1}) \cdot \vec{u_2}$$

où :

- P est le produit mixte des vecteurs $\vec{u_1}$ et $\vec{u_2}$.

Ensuite, on procède à une étape 74 de comparaison de la valeur du produit mixte P à un seuil $S_2$ pour contrôler que la distance $\underline{d}$ est inférieure à une limite prédéterminée. En absence de perturbateur, la valeur du produit mixte doit être proche de zéro. A l'inverse, la présence d'un perturbateur se traduit par une valeur élevée de ce produit mixte P.

Ainsi, si la valeur du produit mixte P est inférieure au seuil prédéterminé $S_2$, alors on procède à l'étape 66. Dans le cas contraire, on procède à l'étape 64.

De nombreux autres modes de réalisation sont possibles. Par exemple, l'émetteur peut avoir plus de trois axes d'émission. Dans ce cas, les émissions de champ magnétique sur les axes supplémentaires au-delà de trois font qu'il y a redondance dans les informations mesurées par les capteurs. Cette redondance peut être utilisée par la suite pour s'affranchir des perturbations causées par le perturbateur magnétique dans des applications telles que la localisation de la position de l'objet 4 par rapport au capteur.

L'émetteur 10 peut émettre le champ magnétique sur chacun de ses axes séquentiellement dans le temps ou en même temps. Dans ce dernier cas, de préférence, les champs seront émis à des fréquences différentes sur chacun des axes pour pouvoir les distinguer les uns des autres du côté capteur.

D'autres formules que celles données ci-dessus peuvent être utilisées pour calculer les coordonnées des vecteurs directeurs. En particulier, les vecteurs directeurs peuvent avoir pour origine le centre géométrique de l'émetteur et pointer vers des capteurs respectifs.

Les axes des repères $R_1$ et $R_2$ ne sont pas nécessairement parallèles. Dans ce cas, un changement de repère est réalisé sur les coordonnées de l'un des vecteurs directeurs $\vec{u_1}$ ou $\vec{u_2}$ afin que les coordonnées de ces deux vecteurs soient exprimées dans le même repère avant de calculer les vecteurs $\vec{n_1}$ et $\vec{n_2}$ ou le produit mixte P.

Le détecteur de perturbateur magnétique peut comporter plus de deux capteurs triaxes.

La vérification que la plus petite distance d entre les axes $L_1$ et $L_2$ est inférieure à une limite prédéterminée peut être réalisée par d'autres méthodes que celles décrites précédemment. Par exemple, il est possible de déterminer l'équation de ces axes dans un même repère, puis de calculer la plus petite distance d entre ces deux axes à partir de ces équations avant de comparer cette distance à la limite prédéterminée.

Ici, le système 2 a été décrit dans le cas particulier où l'émetteur est mobile et les capteurs sont fixes. Toutefois, ce qui a été décrit ici s'applique au cas inverse dans lequel l'émetteur est fixe et les capteurs sont mobiles dans le repère 6.

Il n'est pas nécessaire que les capteurs soient fixes dans le répère 6. Par contre, ce qui est nécessaire, c'est que la position relative de l'émetteur par rapport aux capteurs ne varie pas entre l'instant où l'un des capteurs réalise ses mesures et l'instant où l'autre capteur réalise ses mesures. Par exemple, cette condition est aussi satisfaite à partir du moment où les mesures des capteurs sont réalisées en même temps même si les capteurs se déplacent dans le référentiel.

Si la position des capteurs n'est pas fixe, la position relative d'un capteur par rapport à l'autre doit être connue avant d'exécuter l'un des procédés décrits précédemment. Par position « connue », on signifie qu'au moins la direction ou la norme du vecteur $\vec{D}$ est connue et, de préférence, que sa direction est connue ou que sa direction et sa norme sont également connues. Pour connaître la position relative d'un capteur par rapport à l'autre, de nombreuses méthodes sont possibles. Par exemple, un filtre de Kalman peut être utilisé à cet effet. Plus précisément, ce filtre de Kalman est construit à partir des équations de l'électromagnétisme reliant les champs magnétiques émis aux champs magnétiques mesurés. Dans ces équations, les inconnues sont les positions des capteurs et, éventuellement, la position de l'émetteur.

Dans une variante, seule la direction du vecteur $\vec{D}$ est connue et sa norme est inconnue. Dans cette variante, la norme du vecteur $\vec{D}$ est choisie égale à une valeur arbitraire, par exemple égale à un. Ensuite, une phase d'apprentissage est mise en oeuvre pour régler la valeur du seuil $S_1$ ou $S_2$ au-delà duquel la présence d'un perturbateur est détectée. Par exemple, cette variante est identique au procédé de la figure 2 sauf que seule la direction du vecteur $\vec{D}$ est connue. La valeur des seuils $S_1$ ou $S_2$ peut être indépendante de la norme du vecteur $\vec{D}$, par exemple, si tous les vecteurs utilisés sont normalisés.

Pour simplifier les capteurs 26 et 28, ceux-ci peuvent être formés d'une seule spire chacun.

Les procédés et le système décrits ici peuvent être utilisés dans de nombreux domaines différents d'application autre que le domaine médical. Par exemple, le système peut être utilisé pour localiser un aspirateur dans une pièce ou pour localiser une personne, telle qu'un enfant, porteur d'un émetteur magnétique.

**Revendications**

1. Procédé de détection d'un perturbateur magnétique d'un champ magnétique émis par un émetteur au moins triaxe de champ magnétique, ce procédé comportant :

   - la mesure (52) du champ magnétique émis par l'émetteur par au moins deux capteurs triaxes placés à des positions différentes connues, la position relative de l'émetteur par rapport aux capteurs ne variant pas entre l'instant où l'un des capteurs réalise ses mesures et l'instant où l'autre capteur réalise ses mesures, **caractérisé en ce que** le procédé comporte :
   - pour chaque capteur, la détermination (54), à partir du champ magnétique mesuré par ce capteur, des coordonnées d'un vecteur directeur colinéaire à un axe passant par le centre géométrique de l'émetteur et le centre géométrique de ce capteur, le centre géométrique de l'émetteur étant le point où se situe une source ponctuelle de champ magnétique qui modélise cet émetteur et le capteur étant modélisable par un transducteur ponctuel situé en un point au niveau duquel est mesuré le champ magnétique et constituant le centre géométrique de ce capteur,
   - la vérification (56 ; 70) que la plus petite distance entre les axes colinéaires chacun à l'un des vecteurs directeurs est inférieure à une limite prédéterminée, et
   - dans la négative, le signalement (64) de la présence d'un perturbateur magnétique et, dans le cas contraire, l'absence de ce signalement.

2. Procédé selon la revendication 1, dans lequel la vérification est au moins réalisée pour des premier et second vecteurs directeurs $\vec{u}_1$, $\vec{u}_2$ et comprend :

   - le calcul (58) des coordonnées d'un premier vecteur $\vec{n}_1$ normal au vecteur directeur $\vec{u}_1$ et à un axe $D$ passant par les centres géométriques des deux capteurs utilisés pour déterminer les vecteurs directeurs $\vec{u}_1$ et $\vec{u}_2$,
   - le calcul (58) des coordonnées d'un second vecteur $\vec{n}_2$ normal au vecteur directeur $\vec{u}_2$ et à l'axe D,
   - le calcul (60) de l'angle entre les vecteurs $\vec{n}_1$ et $\vec{n}_2$, et
   - le contrôle (62) que cet angle est inférieur à un seuil correspondant à la limite prédéterminée au-delà de laquelle le signalement du perturbateur magnétique est déclenché.

3. Procédé selon la revendication 1, dans lequel la vérification est au moins réalisée pour des premier et second vecteurs directeurs $\vec{u}_1$, $\vec{u}_2$ et comprend :

   - le calcul (72) de la valeur d'un produit mixte, le produit mixte étant défini par la relation suivante : $(\vec{D} \otimes \vec{u}_1) \cdot \vec{u}_2$, où $\otimes$ et '·' sont, respectivement, les opérations produits vectoriel et scalaire et le vecteur $\vec{D}$ est un vecteur colinéaire à un axe $D$ passant par les centres géométriques des deux capteurs utilisés pour déterminer les vecteurs directeurs $\vec{u}_1$ et $\vec{u}_2$ et
   - le contrôle (74) que la valeur du produit mixte est inférieure à un seuil correspondant à la limite prédéterminée au-delà de laquelle le signalement du perturbateur magnétique est déclenché.

4. Procédé selon l'une quelconque des revendications précédentes, dans lequel les positions des capteurs, dans un même repère, sont fixes.

5. Procédé de localisation d'un objet dans un repère à l'aide d'au moins un émetteur au moins triaxe de champ magnétique et d'au moins deux capteurs triaxes du champ magnétique émis par l'émetteur, chaque capteur étant solidaire du repère et chaque émetteur étant solidaire de l'objet ou vice versa, ce procédé comportant :

- l'émission (50) d'un champ magnétique par l'émetteur et la mesure (52) de ce champ magnétique par les capteurs,
- la localisation (66) de l'objet dans le repère à partir des mesures réalisées par les capteurs,

**caractérisé en ce que** ce procédé comprend également la détection (54, 56 ; 54, 70) d'un perturbateur du champ magnétique émis par l'émetteur en utilisant les mêmes capteurs que lors de l'étape de localisation et en mettant en oeuvre un procédé conforme à l'une quelconque des revendications précédentes.

6. Support (46) d'enregistrement d'informations, **caractérisé en ce qu'**il comporte des instructions pour la mise en oeuvre d'un procédé conforme à l'une quelconque des revendications précédentes, lorsque ces instructions sont exécutées par un calculateur électronique.

7. Détecteur d'un perturbateur magnétique d'un champ magnétique émis par un émetteur au moins triaxe de champ magnétique, ce détecteur comportant :

- au moins deux capteurs triaxes (26, 28) aptes à mesurer le champ magnétique émis par l'émetteur, ces capteurs étant placés à des positions différentes connues, et
- une unité (24) de traitement raccordée aux capteurs pour traiter les mesures du champ magnétique de l'émetteur,

**caractérisé en ce que** cette unité de traitement est apte :
- pour chaque capteur, à déterminer, à partir du champ magnétique mesuré par ce capteur, des coordonnées d'un vecteur directeur colinéaire à un axe passant par le centre géométrique de l'émetteur et le centre géométrique de ce capteur, le centre géométrique de l'émetteur étant le point où se situe une source ponctuelle de champ magnétique qui modélise cet émetteur et le capteur étant modélisable par un transducteur ponctuel situé en un point au niveau duquel est mesuré le champ magnétique et constituant le centre géométrique de ce capteur,
- à vérifier que la plus petite distance entre les axes colinéaires chacun à un des vecteurs directeurs est inférieure à une limite prédéterminée, et
- dans la négative, à signaler la présence d'un perturbateur magnétique et, dans le cas contraire, à ne pas signaler la présence de ce perturbateur magnétique.

8. Détecteur selon la revendication 7, dans lequel les positions des capteurs, dans un même repère, sont fixes.

9. Système de localisation d'un objet dans un repère, ce système comportant :

- au moins un émetteur (10) au moins triaxe de champ magnétique,
- au moins deux capteurs triaxes (26, 28) pour mesurer le champ magnétique émis par cet émetteur, les capteurs étant fixes dans le repère et l'émetteur ou les émetteurs étant solidaires de l'objet ou vice versa,
- un module (42) de localisation apte à localiser l'objet dans le repère à partir des mesures réalisées par les capteurs,

**caractérisé en ce que** le système comprend un détecteur (26, 28, 42) d'un perturbateur du champ magnétique émis par l'émetteur conforme à la revendication 7 ou 8, les capteurs de ce détecteur étant communs avec ceux utilisés par le module de localisation.

## Claims

1. Method for detecting a magnetic disturber of a magnetic field emitted by an at least triaxial emitter of a magnetic field, this method comprising:

- measuring (52) the magnetic field emitted by the emitter using at least two triaxial sensors placed in different known positions, the position of the emitter relative to the sensors not varying between the moment at which one of the sensors makes its measurements and the moment in which the other sensor makes its measurements, **characterised in that** the method comprises:
- for each sensor, determining (54) from the magnetic field measured by this sensor the coordinates of a directing vector collinear with an axis passing through the geometrical centre of the emitter and the geometrical centre of this sensor, the geometrical centre of the emitter being the point at which there is a magnetic-field point source that models this emitter, and the sensor being able to be modelled by a point transducer situated at a point at which the magnetic field is measured and forming the geometric centre of this sensor,

- verifying (56; 70) that the shortest distance between the axes, each of which is collinear with one of the directing vectors, is shorter than a predetermined limit, and

- if it is not, indicating (64) the presence of a magnetic disturber and, in the contrary case, the absence of this indication.

2. Method according to Claim 1, wherein the verification is at least carried out for first and second directing vectors $u_1$, $u_2$ and comprises:

- computing (58) the coordinates of a first vector $n_1$ normal to the directing vector $u_1$ and to an axis $D$ passing through the geometrical centres of the two sensors used to determine the directing vectors $u_1$ and $u_2$,
- computing (58) the coordinates of a second vector $n_2$ normal to the directing vector $u_2$ and to the axis D,
- computing (60) the angle between the vectors $n_1$ and $n_2$, and
- checking (62) that this angle is below a threshold corresponding to the predetermined limit beyond which the indication of the magnetic disturber is initiated.

3. Method according to Claim 1, wherein the verification is at least carried out for first and second directing vectors $u_1$, $u_2$ and comprises:

- computing (72) the value of a mixed product, the mixed product being defined by the following relation: ($D \otimes u_1$)$\cdot u_2$, where $\otimes$ and '$\cdot$' are respectively the vector and scalar product operations and the vector $D$ is a vector collinear with an axis $D$ passing through the geometrical centres of the two sensors used to determine the directing vectors $u_1$ and $u_2$, and
- checking (74) that the value of the mixed product is below a threshold corresponding to the predetermined limit beyond which the indication of the magnetic disturber is initiated.

4. Method according to any one of the preceding claims, wherein the positions of the sensors, in one and the same reference frame, are fixed.

5. Method for locating an object in a reference frame with the aid of at least one magnetic field emitter that is at least triaxial and with at least two triaxial sensors of the magnetic field emitted by the emitter, each sensor being secured to the reference frame and each emitter being secured to the object or vice versa, this method comprising:

- the emission (50) of a magnetic field via the emitter and the measurement (52) of this magnetic field by the sensors,
- the location (66) of the object in the reference frame from the measurements made by the sensors, **characterised in that** this method also comprises the detection (54, 56; 54, 70) of a disturber of the magnetic field emitted by the emitter by using the same sensors as during the location step and by applying a method according to any one of the preceding claims.

6. Information recording medium (46), **characterised in that** it comprises instructions for the implementation of a method according to any one of the preceding claims, when these instructions are executed by an electronic computer.

7. Detector of a magnetic disturber of a magnetic field emitted by an at least triaxial emitter of a magnetic field, this detector comprising:

- at least two triaxial sensors (26, 28) capable of measuring the magnetic field emitted by the emitter, these sensors being placed in different known positions, and
- a processing unit (24) connected to the sensors in order to process the measurements of the magnetic field of the emitter,
**characterised in that** this processing unit is capable:
- for each sensor, of determining, from the magnetic field measured by this sensor, coordinates of a directing vector collinear with an axis passing through the geometrical centre of the emitter and the geometrical centre of this sensor, the geometrical centre of the emitter being the point at which there is a magnetic-field point source which models this emitter and the sensor being able to be modelled by a point transducer situated at a point at which the magnetic field is measured and forming the geometrical centre of this sensor,
- of verifying that the shortest distance between the axes, each of which is collinear with one of the directing vectors, is shorter than a predetermined limit, and
- if it is not, of indicating the presence of a magnetic disturber and, in the contrary case, of not indicating the presence of this magnetic disturber.

**8.** Detector according to Claim 7, wherein the positions of the sensors, in one and the same reference frame, are fixed.

**9.** System for locating an object in a reference frame, this system comprising:

- at least one at least triaxial emitter (10) of a magnetic field,
- at least two triaxial sensors (26, 28) for measuring the magnetic field emitted by this emitter, the sensors being fixed in the reference frame and the emitter or the emitters being secured to the object or vice versa,
- a location module (42) capable of locating the object in the reference frame from measurements made by the sensors,

**characterised in that** the system comprises a detector (26, 28, 42) of a disturber of the magnetic field emitted by the emitter according to Claim 7 or 8, the sensors of this detector being the same as those used by the location module.

## Patentansprüche

**1.** Verfahren zur Erkennung eines magnetischen Störelements eines Magnetfeldes, das von einem mindestens dreiachsigen Magnetfeldsender ausgesendet wird, wobei dieses Verfahren Folgendes aufweist:

- die Messung (52) des von dem Sender ausgesendeten Magnetfeldes durch mindestens zwei dreiachsige Sensoren, die an verschiedenen bekannten Positionen angeordnet sind, wobei sich die relative Position des Senders bezüglich der Sensoren zwischen dem Zeitpunkt, zu dem der eine der Sensoren seine Messungen durchführt, und dem Zeitpunkt, zu dem der andere Sensor seine Messungen durchführt, nicht ändert,
**dadurch gekennzeichnet, dass** das Verfahren Folgendes aufweist:
- für jeden Sensor, die Bestimmung (54), ausgehend von dem durch diesen Sensor gemessenen Magnetfeld, der Koordinaten eines Richtungsvektors, der zu einer Achse kollinear ist, die durch den geometrischen Mittelpunkt des Senders und den geometrischen Mittelpunkt dieses Sensors verläuft, wobei der geometrische Mittelpunkt des Senders der Punkt ist, an dem sich eine punktförmige Magnetfeldquelle befindet, welche diesen Sender modelliert, und wobei der Sensor durch einen punktförmigen Messwandler modellierbar ist, der sich in einem Punkt befindet, an dem das Magnetfeld gemessen wird und der den geometrischen Mittelpunkt dieses Sensors bildet,
- die Überprüfung (56; 70), ob der kleinste Abstand zwischen den Achsen, die jeweils zu einem der Richtungsvektoren kollinear sind, kleiner als ein vorgegebener Grenzwert ist, und
- falls nein, die Meldung (64) des Vorhandenseins eines magnetischen Störelements, und im entgegengesetzten Fall das Ausbleiben dieser Meldung.

**2.** Verfahren nach Anspruch 1, wobei die Überprüfung mindestens für einen ersten und einen zweiten Richtungsvektor $\vec{u}_1$, $\vec{u}_2$ durchgeführt wird und Folgendes beinhaltet:

- die Berechnung (58) der Koordinaten eines ersten Vektors $\vec{n}_1$ der zu dem Richtungsvektor $\vec{u}_1$ und zu einer Achse $D$, die durch die geometrischen Mittelpunkte der beiden Sensoren verläuft, die zur Bestimmung der Richtungsvektoren $\vec{u}_1$ und $\vec{u}_2$ verwendet wurden, normal ist,
- die Berechnung (58) der Koordinaten eines zweiten Vektors $\vec{n}_2$ der zu dem Richtungsvektor $\vec{u}_2$ und zu der Achse $D$ normal ist,
- die Berechnung (60) des Winkels zwischen den Vektoren $\vec{n}_1$ und $\vec{n}_2$ und
- die Kontrolle (62), ob dieser Winkel kleiner als ein Schwellenwert ist, der dem vorgegebenen Grenzwert entspricht, bei dessen Überschreitung die Meldung des magnetischen Störelements ausgelöst wird.

**3.** Verfahren nach Anspruch 1, wobei die Überprüfung mindestens für einen ersten und einen zweiten Richtungsvektor $\vec{u}_1$, $\vec{u}_2$ durchgeführt wird und Folgendes beinhaltet:

- die Berechnung (72) des Wertes eines gemischten Produktes, wobei das gemischte Produkt durch die folgende Beziehung definiert ist: $(\vec{D} \otimes \vec{u}_1) \cdot \vec{u}_2$ , wobei $\otimes$ und '·' die Operationen Vektorprodukt bzw. Skalarprodukt sind und der Vektor $\vec{D}$ ein Vektor ist, der zu einer Achse $D$ kollinear ist, die durch die geometrischen Mittelpunkte der beiden Sensoren verläuft, die zur Bestimmung der Richtungsvektoren $\vec{u}_1$ und $\vec{u}_2$ verwendet wurden, und

- die Kontrolle (74), ob der Wert des gemischten Produktes kleiner als ein Schwellenwert ist, der dem vorgegebenen Grenzwert entspricht, bei dessen Überschreitung die Meldung des magnetischen Störelements ausgelöst wird.

4. Verfahren nach einem der vorhergehenden Ansprüche, wobei die Positionen der Sensoren in ein und demselben Koordinatensystem fest sind.

5. Verfahren zur Lokalisierung eines Objekts in einem Koordinatensystem mit Hilfe mindestens eines mindestens dreiachsigen Magnetfeldsenders und mindestens zweier dreiachsiger Sensoren des von dem Sender ausgesendeten Magnetfeldes, wobei jeder Sensor mit dem Koordinatensystem fest verbunden ist und jeder Sender mit dem Objekt fest verbunden ist, oder umgekehrt, wobei dieses Verfahren Folgendes aufweist:

- das Aussenden (50) eines Magnetfeldes durch den Sender und die Messung (52) dieses Magnetfeldes durch die Sensoren,
- die Lokalisierung (66) des Objekts in dem Koordinatensystem ausgehend von den Messungen, die durch die Sensoren durchgeführt wurden,

**dadurch gekennzeichnet, dass** dieses Verfahren außerdem die Erkennung (54, 56; 54, 70) eines Störelements des von dem Sender ausgesendeten Magnetfeldes unter Verwendung derselben Sensoren wie im Schritt der Lokalisierung und unter Durchführung eines Verfahrens nach einem der vorhergehenden Ansprüche aufweist.

6. Informationsaufzeichnungsmedium (46), **dadurch gekennzeichnet, dass** es Anweisungen für die Durchführung eines Verfahrens nach einem der vorhergehenden Ansprüche, wenn diese Anweisungen durch einen Computer ausgeführt werden, aufweist.

7. Detektor für ein magnetisches Störelement eines Magnetfeldes, das von einem mindestens dreiachsigen Magnetfeldsender ausgesendet wird, wobei dieser Detektor Folgendes aufweist:

- mindestens zwei dreiachsige Sensoren (26, 28), die geeignet sind, das von dem Sender ausgesendete Magnetfeld zu messen, wobei diese Sensoren an verschiedenen bekannten Positionen angeordnet sind, und
- eine Verarbeitungseinheit (24), die an die Sensoren angeschlossen ist, zum Verarbeiten der Messungen des Magnetfeldes des Senders,

**dadurch gekennzeichnet, dass** diese Verarbeitungseinheit geeignet ist:
- für jeden Sensor, ausgehend von dem durch diesen Sensor gemessenen Magnetfeld, Koordinaten eines Richtungsvektors zu bestimmen, der zu einer Achse kollinear ist, die durch den geometrischen Mittelpunkt des Senders und den geometrischen Mittelpunkt dieses Sensors verläuft, wobei der geometrische Mittelpunkt des Senders der Punkt ist, an dem sich eine punktförmige Magnetfeldquelle befindet, welche diesen Sender modelliert, und wobei der Sensor durch einen punktförmigen Messwandler modellierbar ist, der sich in einem Punkt befindet, an dem das Magnetfeld gemessen wird und der den geometrischen Mittelpunkt dieses Sensors bildet,
- zu überprüfen, ob der kleinste Abstand zwischen den Achsen, die jeweils zu einem der Richtungsvektoren kollinear sind, kleiner als ein vorgegebener Grenzwert ist, und
- falls nein, das Vorhandensein eines magnetischen Störelements zu melden, und im entgegengesetzten Fall das Vorhandensein dieses magnetischen Störelements nicht zu melden.

8. Detektor nach Anspruch 7, wobei die Positionen der Sensoren in ein und demselben Koordinatensystem fest sind.

9. System zur Lokalisierung eines Objekts in einem Koordinatensystem, wobei dieses System Folgendes aufweist:

- mindestens einen mindestens dreiachsigen Magnetfeldsender (10),
- mindestens zwei dreiachsige Sensoren (26, 28) zum Messen des von diesem Sender ausgesendeten Magnetfeldes, wobei die Sensoren in dem Koordinatensystem feststehend sind und der Sender oder die Sender mit dem Objekt fest verbunden sind, oder umgekehrt,

- ein Lokalisierungsmodul (42), das geeignet ist, das Objekt in dem Koordinatensystem ausgehend von den Messungen, die durch die Sensoren durchgeführt wurden, zu lokalisieren,

**dadurch gekennzeichnet, dass** das System einen Detektor (26, 28, 42) für ein Störelement des von dem Sender ausgesendeten Magnetfeldes nach Anspruch 7 oder 8 aufweist, wobei die Sensoren dieses Detektors mit denjenigen, die durch das Lokalisierungsmodul verwendet werden, zusammengeschlossen sind.

Fig.1

EP 2 311 370 B1

Fig.2

Fig.3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1502544 A
- EP 0993804 A
- US 6841994 B1